# EUROPEAN PATENT APPLICATION

(11) **EP 1 323 388 A1**
(43) Date of publication of application: **02.07.2003**
(21) Application number: 02011812.1
(22) Date of filing: 28.05.2002
(51) Int. Cl.: A61B 17/20, A61M 5/00, B65D 85/24

(54) **Medical needle assemblies**

(30) Priority: 28.12.2001 US 344304 P; 09.05.2002 US 141538
(71) Applicant: Becton, Dickinson and Company, Franklin Lakes, New Jersey 07417 (US)
(72) Inventor: Sprieck, Terry L., Columbus, NB 68601 (US); Prais, Alfred Wesley, Hewitt, NJ 07421 (US); Alchas, Paul G., Wayne, NJ 07470 (US)
(74) Representative: Selting, Günther, Dipl.-Ing.

(57) **Abstract**

A shielded, sterile, single-use unit dose needle assembly (10) includes a unit dose needle (12) with a hub (22) and a packaging shield (40). The unit dose needle (12) has a handle end (14) and a prong end (16) configured to hold a unit dose of vaccine. The hub (22) is fixedly attached to the handle end (14) of the unit dose needle (12) and includes a tapered mating surface (32). The packaging shield (40) includes a tubular housing (42) having an open end (44) and a closed end (46) with an internal opening (48) extending therebetween. The open end (44) of the packaging shield (40) can be removably attached to the tapered mating surface (32) of the hub (22) to form an air-tight seal, with the unit dose needle (12) contained within the internal opening (48).

## Description

### CROSS-REFERENCE TO RELATED APPLICATIONS

This application claims priority from U.S. Provisional Patent Application Serial. No. 60/344,304, filed December 28, 2001, and entitled "Bifurcated Needle Assembly."

### BACKGROUND OF THE INVENTION

### 1. Field of the Invention

The present invention relates to needles for use in medical procedures and in particular to safety shielded needles and needle assemblies for use in medical procedures.

### 2. Description of Related Art

Bifurcated or forked end needles are well-known for providing a simple and effective means for a doctor to administer a vaccine. During use, the bifurcated tip of the bifurcated needle is put into contact with either a dried or liquid substance, which adheres to the bifurcated needle tip. The bifurcated needle tip is then put into contact with the skin of the patient who is being administered the vaccination. The skin is either scratched or pierced with the needle tip so that the vaccination material may be absorbed into the skin of the patient. An alternative method of delivering the vaccination includes placing a drop of the vaccine onto the skin of the patient and contacting the skin of the patient with the bifurcated needle tip through the drop of vaccination. Alternatively, a standard pointed needle tip may also be used when the drop of vaccination is applied directly to the skin of the patient.

The bifurcated needle is considered a significant medical advancement because it has allowed more people to be vaccinated with less serum. This has been especially important for those living in less developed areas because of the efficient and easy to use design, as well as the ease of replication.

Vaccination effectiveness, however, is reduced if the bifurcated needle is reused too many times. Moreover, reuse of such vaccination needles exposes patients to the risk of transmission of infectious diseases through percutaneous contact through the skin. Additionally, medical care workers using traditional vaccination needles are at an increased risk of exposure to infectious diseases due to the design of such needles, which makes them difficult to handle, as well as due to the repeated use of such needles.

In particular, bifurcated needles used to administer vaccinations are not traditionally sterilized or packaged in a single-use container that would enable convenient storage and subsequent use. Additionally, such needles have traditionally been difficult to handle in that they typically do not include a hub attached to the opposite end of a needle from the tip, and do not typically include any sort of shield for protection from the needle prior to and during use.

For example, U.S. Patent No. 3,194,237 to Rubin discloses a vaccinating needle having a main shank with a pair of prongs at one end that define a slot of predetermined length, width and depth therebetween to hold an amount of liquid by capillary action. The shank of the needle is of sufficient length so that the non-prong end will function as a handle. U.S. Patent No. 3,948,261 to Steiner discloses a reusable unit dose container for vaccines contained within a rigid receptacle, with a compressible closure for supporting a bifurcated needle bearing dried vaccine. The closure is adapted to support the needle in the container during a lyophilizing process while liquid vaccine is dried on the needle. The closure has grooves which permit the vaporized liquid from the vaccine to be withdrawn from the receptacle during lyophilizing, and can further seal the container.

There exists a need for a safety assembly for use with a unit dose vaccination needle that is easily manufactured, that is simple to use, that is easily sterilized and maintained in a sterile condition until used, that can be safely disposed of, and that does not interfere with normal practices of bifurcated needle use.

### SUMMARY OF THE INVENTION

The present invention is directed a shielded, sterile, single-use needle assembly for administering a unit dose of a vaccine. The assembly includes a unit dose needle with a hub and a packaging shield. The unit dose needle has a prong end configured to hold a unit dose of a vaccine and a handle end. The hub is fixedly attached to the handle end of the unit dose needle and includes a tapered mating surface. The packaging shield includes a tubular housing having an open end and a closed end with an internal opening extending therebetween. The open end of the packaging shield can be removably attached to the tapered mating surface of the hub to form an air-tight seal, with the unit dose needle contained within the internal opening. Desirably, the hub includes means for attaching the assembly to a medical device, such as a standard needle cover.

The present invention is further directed to a method of packaging the present single-use unit dose needle assembly. The packaging method includes providing a sheet of top web material, providing a sheet of bottom web material, and providing a plurality of the present single-use unit dose needle assemblies. The top web material includes a plurality of rows, each row including a plurality of covers for a blister pack, each cover including a perimeter area. The bottom web material includes a plurality of rows, each row including a plurality of bottoms for a blister pack, each bottom including a perimeter area and a pocket. The single-use unit dose needle assemblies are placed in each of the pockets in the bottom web material, which are then sealed joining the perimeter areas in the top web material to the perimeter areas in the bottom web material to form a plurality of sealed single-use unit dose needle assembly packages.

### BRIEF DESCRIPTION OF THE DRAWINGS

FIG. 1 is a perspective view of a shielded, sterile single-use needle assembly of the present invention;

FIG. 2 is a side cross section view of the needle assembly shown in FIG. 1;

FIG. 3 is an exploded perspective view of the needle assembly of FIG. 1;

FIG. 4 is a perspective view of a needle assembly in accordance with an alternate embodiment of the present invention;

FIG. 5 is an exploded perspective view of a packaged needle assembly of the present invention;

FIG. 6 is an exploded perspective view demonstrating packaging of the packaged needle assembly of the present invention;

FIG. 7 is an exploded perspective view of the needle assembly of the present invention packaged in a plurality of strips of blister packages and a carton;

FIG. 8 is a perspective view of a plurality of cartons and a case as packaged;

FIG. 9 is a perspective view of a sealed case;

FIG. 10 is a perspective view of a plurality of cases shrink wrapped on a pallet; and

FIG. 11 is a perspective view of two stacked shrink wrapped pallets of cases.

### DETAILED DESCRIPTION OF THE PREFERRED EMBODIMENTS

In the following description and accompanying drawings, like reference numbers, as used in the various figures, refer to like features or elements. The terms top and bottom, as used herein, refer to the orientation of a given element as shown in the drawings.

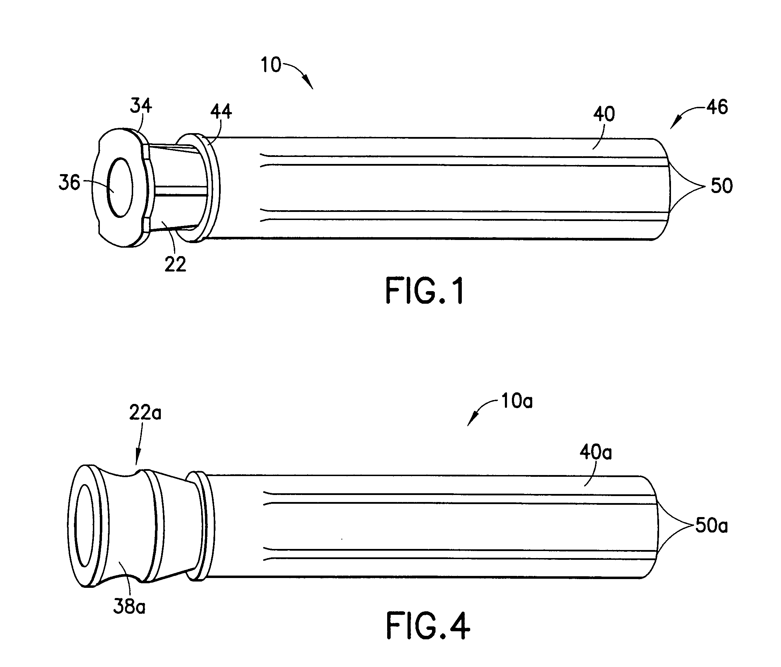

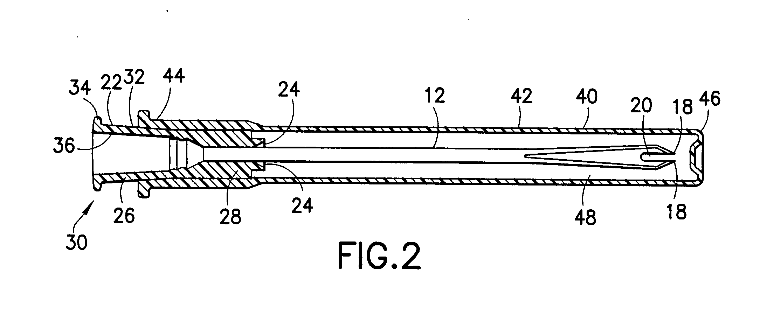

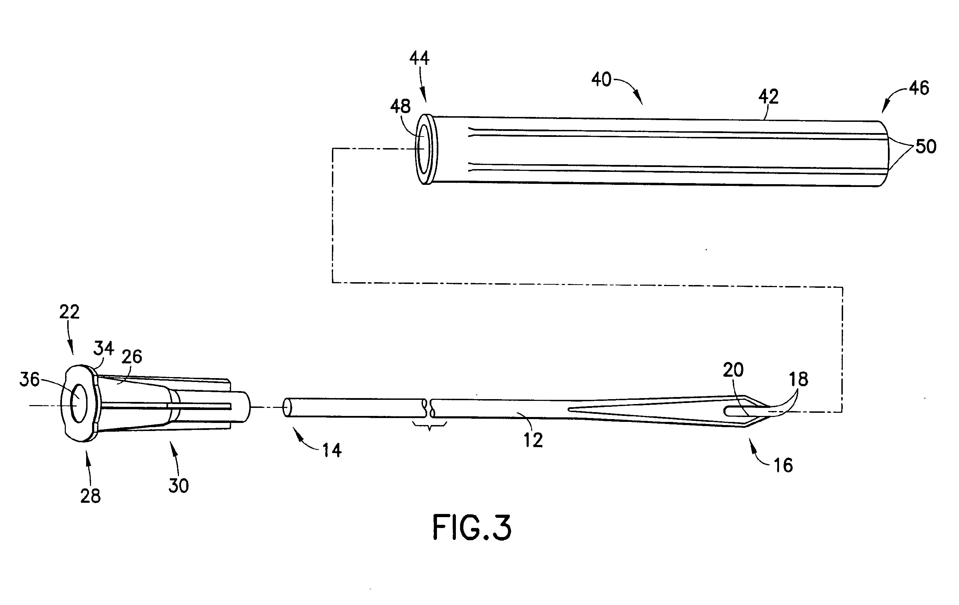

Referring to the drawings in which like reference characters refer to like parts throughout the several views thereof, FIGS. 1-3 depict a shielded, sterile, single-use unit dose needle assembly **10** in accordance with the present invention and the related features. The unit dose needle assembly **10** is intended for use for the administration of vaccines applied to or through the skin of the patient, and is intended as a single-use vaccination needle assembly including features to maintain sterility of the needle during packaging and to provide ease of use for the medical practitioner, as will be described in more detail herein.

The unit dose needle assembly **10** generally includes a unit dose needle for administering a unit dose of a vaccine, such as bifurcated needle **12**, which is supported by a hub **22,** and enclosed within a needle shield **40.** While unit dose needle assembly **10** is described herein in terms of a preferred embodiment including bifurcated needle **12,** unit dose needle assembly **10** may include any unit dose needle capable of administering a unit dose of a vaccine, such as in a dry powder or liquid form, as is well known in the art.

Bifurcated needle **12** includes a handle end or a non-patient end at proximal end **14,** and an opposed prong end or patient end at distal end **16.** Bifurcated needle **12** is provided with two sharp prongs **18** positioned at a distal end **16** of the needle. The prongs **18** are separated by a U-shaped channel **20** configured to hold a unit dose of vaccine. The prongs **18** are intended to penetrate or abrade the skin of the patient to administer the vaccine disposed in the U-shaped channel **20.** Bifurcated needle **12** may be constructed of any material known in the art, such as metal or plastic, and is desirably constructed of a medical grade surgical steel.

Hub **22** is fixedly attached to the proximal end **14** of bifurcated needle **12,** such as through an adhesive joint **24.** Adhesive joint **24** may be provided through any adhesive capable of fixedly attaching or adhering bifurcated needle **12** to hub **22,** such as an epoxy or equivalent adhesive. Hub **22** includes a hub housing **26** including a proximal end **28** and a distal end **30,** with the external surface of hub housing **26** defining an outer tapered surface **32** extending therealong. Desirably, distal end **30** of hub **22** includes an internal bore having an internal diameter of approximately the same size as the outer diameter of the proximal end **14** of bifurcated needle **12,** for accommodating and fixedly adhering bifurcated needle **12** within such an internal bore of hub **22.**

The hub **22** may further include means for attachment with a medical device, such as luer lugs **34** extending at the proximal end thereof, with an internal luer taper **36** which extends internally within a portion of hub housing **26.** Internal luer taper **36** and luer lugs **34** are designed to engage a medical device, such as a hypodermic syringe or a standard needle holder, for particularly desired uses of the unit dose needle assembly. Such attached medical devices can act as a handle portion for the assembly, thereby facilitating ease of use of the assembly. In addition, such means for attachment may accommodate attachment of a safety shield which can be used to shield the needle after use thereof. Threads (not shown) may further be provided within internal luer taper **36**, for establishing threaded engagement with a corresponding threaded surface of a medical device.

Unit dose needle assembly **10** further includes a needle shield **40** extending about bifurcated needle **12.** Needle shield **40** is of a generally tubular hollow construction, including tubular shield housing **42** extending between a proximal end **44** and a distal end **46,** with the tubular shape of shield housing **42** forming an internal opening **48** extending through needle shield **40.** Proximal end **44** of needle shield **40** is generally open ended, forming a passage for access to internal opening **48,** while distal end **46** is closed ended, forming a wall. Needle shield **40** extends about bifurcated needle **12,** thereby containing bifurcated needle **12** within internal opening **48.** Proximal end **44** may further be provided with a lip extending circumferentially about the open ended passage. Proximal end **44** removably engages the hub **22** along the tapered surface **32** to form an air-tight seal, completely concealing the bifurcated needle **12** and associated prongs **18** therein in a sterile, air-tight manner.

The needle shield **40** serves to protect the bifurcated needle **12** from damage and exposure to soils or other contaminants during shipping and storage, and prior to use of the unit dose needle assembly. The needle shield **40** also provides protection to personnel from needle sticks prior to removing the needle shield **40** for use.

The hub **22** and needle shield **40** may be constructed of any material, and are desirably constructed of a moldable plastic material. Suitable moldable plastics include, but are not limited to polyethylenes, polypropylenes, polyamides, polyesters and fluorinated polyethylenes. Preferably, hub **22** is constructed of a rigid material, while needle shield **40** may be formed from a non-rigid material. By non-rigid material, what is meant is a material that is sufficiently flexible to conform to the tapered surface **32** of the hub **22** to form an air-tight seal. While needle shield **40** may be desirably formed from polypropylene, any material known by those of skill in the art to facilitate sterilization of the unit dose needle **10** may also be used. Needle shield **40** may further include external ribs **50** integrally molded with shield housing **42** and extending longitudinally along the outer surface of shield housing **42** between proximal end **44** and distal end **46.** Such external ribs **50** provide further structural integrity to needle shield **40,** which is particularly useful during packaging and storage.

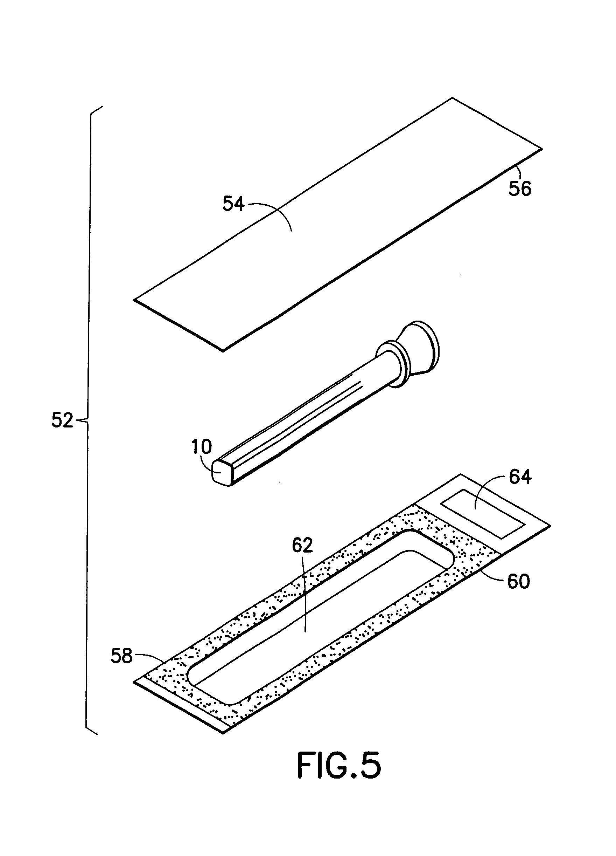

FIG. 4 depicts a further embodiment of the present invention, in which the hub may also include a profile for accommodating a user's fingers. FIG. 4 includes many components which are substantially identical to the components of FIGS. 1-3. Accordingly, similar components are numbered identically to those of FIGS. 1-3, except that a suffix "a" will be used to identify those components in FIG. 4.

As shown in FIG. 4, hub **22a** may include a profile for accommodating a user's fingers, such as arcuate surface **38a** which extends circumferentially about hub **22a.** Such a profile extends from the proximal end **28** of hub **22**, thereby providing ease of use with a user's fingers, and providing a significant surface for a user to grasp during removal of needle shield **40** for exposure of the bifurcated needle **12,** as well as during use of bifurcated needle **12** during administration of the vaccine.

The vaccine may be in any suitable physical form. Suitable physical forms for the vaccine include, but are not limited to liquids, such as solutions, emulsions, and dispersions, or dry powders. Typically, the vaccine will be in a liquid form. Moreover, the vaccine is desirably associated with unit dose needle assembly **10** during storage, and may therefore be contained within the U-shaped channel **20** prior to removal of needle shield **40**. Alternatively, the vaccine may be provided as a separate component, with bifurcated needle **12** being contacted with the vaccine after removing needle shield **40** therefrom just prior to use of the needle for administration of the vaccine.

As noted, needle shield **40** sealingly mates with hub **22** to provide an air-tight connection therebetween, with bifurcated needle **12** contained within the air-tight environment in internal opening **48** of needle shield **40.** Such an air-tight arrangement provides unit dose needle assembly **10** as a self-contained assembly, in the form of a complete, shielded, sterile, single-use unit dose needle assembly, which can be shipped in this form. Alternatively, this unit dose needle assembly **10** may be further packaged to provide additional sterility to the assembly.

For example, referring to FIG. 5, a packaged needle assembly **52** is shown. In the packaged assembly **52,** a blister-type package including a top web **54** having a perimeter edge **56** and a bottom web **58** having a perimeter edge **60** and a pocket **62** suitable for receiving the unit dose needle assembly **10** therein. The perimeter edge **56** of the top web **54** and the perimeter edge **60** of the bottom web **58** are sealed together along their respective lengths. Thus, the unit dose needle assembly **10** may be sealed between the top web **54** and the bottom web **58** to provide further sterility to the unit dose needle assembly **10**. In this manner, a blister-type packaged assembly **52** conceals the unit dose needle assembly **10** in a sterile environment. The bottom web **58** may include an embossing area **64** capable of receiving printed images such as lot numbers or other identifying information. The top web **54** and the bottom web **58** may comprise any suitable material. Suitable materials include, but are not limited to paper and polymer films, and combinations thereof.

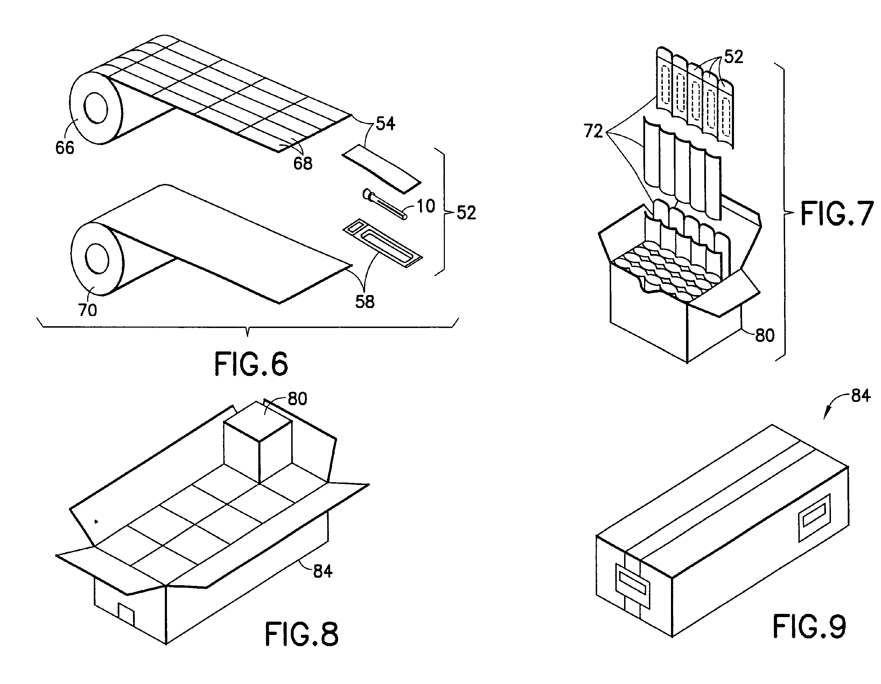

Packaging of unit dose needle assembly **10** within packaged assembly **52** may be accomplished through any known packaging technique. For example, as shown in FIG. 6, packaging of the unit dose needle assembly **10** may include providing a sheet of top web material **66,** where the top web material **66** includes a plurality of rows **68,** each row including a plurality of individual units forming individual covers in the form of top web **54** of packaged assembly **52.** Each of the covers of top web **54** includes a perimeter edge **56**. A roll of bottom web material **70** is further provided, where the bottom web material **70** includes a plurality of rows, each row including a plurality of bottom webs **58** for the blister pack of packaged assembly **52**. Each of the bottom webs **58** includes a perimeter edge **60** and a pocket **62.** The pocket **62** is adapted to receive the unit dose needle assembly **10**.

The top web **54** is formed from a roll of top material **66** in a sectionalized fashion. Likewise, the bottom web **58** may be formed from a roll of bottom material **70** in a similar manner. During production, one unit dose needle assembly **10** is inserted into each pocket **62** and the rolls of material **66, 70** are mated so that a plurality of assemblies **52** may be interconnected between the rolls of material **66, 70.** The perimeter edges **56** in the top web material **54** are sealed to the perimeter edges **60** in the bottom web material **58** to form a plurality of sealed packaged assemblies **52.**

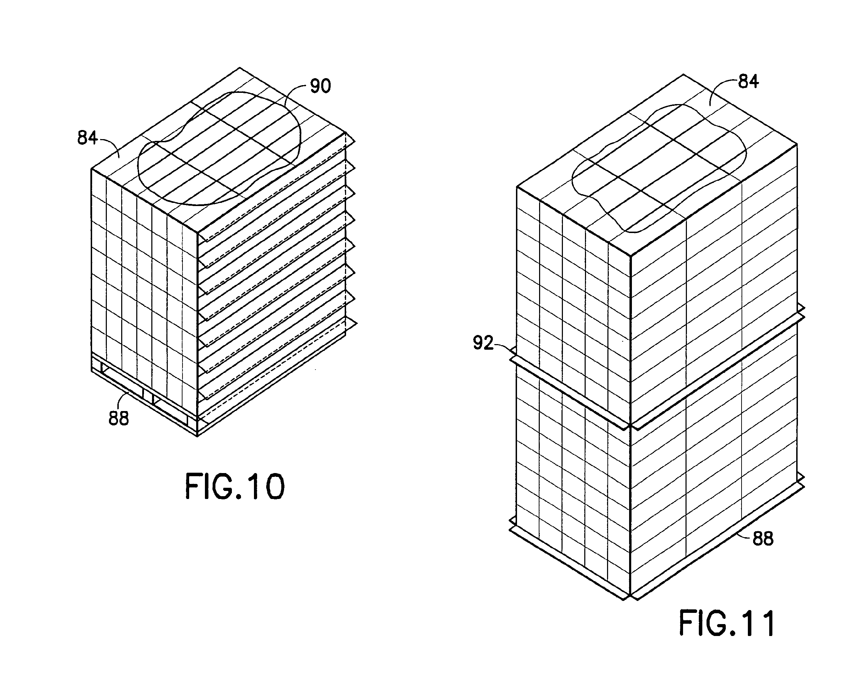

Turning to FIGS. 7-11, a plurality of the blister packaged assemblies **52** may be removed from the interconnected rolls so that a strip **72** comprising a row of packaged assemblies **52** may be packaged in a carton **80** to provide a packaged carton **82.** Strips **72** of packaged assemblies **52** may optionally be perforated so that a single blister package **60** may be removed from the strip **72** when needed for use. A plurality of strips **72** may be placed in a single carton **80** for shipment to an end use destination. A plurality of packaged cartons **80** may be placed in a case **84** (FIG. 8) that may be taped and labeled for shipping (FIG. 9). A plurality of shipping cases **84** may be secured to a pallet **88,** as shown in FIG. 10. Stretch wrap **90** or an equivalent may be wrapped around the plurality of shipping cases **84** on pallet **88** for further sterilization and shipment.

A plurality of pallets **88** may be stacked upon each other for shipment in bulk as shown in FIG. 11. Each pallet **88** preferably is separated by a slip sheet **92** to prevent the pallets **88** from sliding upon one another during shipment.

While the present invention is satisfied by embodiments in many different forms, there is shown in the drawings and described herein in detail, the preferred embodiments of the invention, with the understanding that the present disclosure is to be considered as exemplary of the principles of the invention and is not intended to limit the invention to the embodiments illustrated. Various other embodiments will be apparent to and readily made by those skilled in the art without departing from the scope and spirit of the invention. The scope of the invention will be measured by the appended claims and their equivalents.

## Claims

1. A shielded, sterile, single-use needle assembly for administering a unit dose of a vaccine comprising:
a unit dose needle having a handle end and a prong end configured to hold a unit dose of a vaccine;
a hub fixedly attached to the handle end of the unit dose needle and including a tapered mating surface; and
a packaging shield comprising a tubular housing having an open end and a closed end with an internal opening extending therebetween, the open end of the packaging shield being removably attached to the tapered mating surface of the hub to form an air-tight seal, with the unit dose needle contained within the internal opening.

2. The needle assembly of claim 1, wherein the unit dose needle comprises a bifurcated needle, wherein the prong end includes two pointed prongs which are capable of penetrating or abrading the skin of a patient, and wherein the prongs are separated by a U-shaped channel capable of holding the unit dose of a vaccine.

3. The needle assembly of claim 1, wherein the unit dose of a vaccine is a liquid.

4. The needle assembly of claim 1, wherein the handle end is fixedly attached to the hub by an adhesive joint.

5. The needle assembly of claim 4, wherein the adhesive joint comprises an epoxy adhesive.

6. The needle assembly of claim 14, wherein the hub includes means for attachment to a medical device.

7. The needle assembly of claim 6, wherein the hub includes an internal luer taper.

8. The needle assembly of claim 1, wherein the hub includes a profile for accommodating a user's fingers.

9. The needle assembly of claim 1, wherein the shield comprises one or more moldable plastics.

10. The needle assembly of claim 9, wherein the plastics are one or more selected from the group consisting of polyethylenes, polypropylenes, polyamides, polyesters and fluorinated polyethylenes.

11. The needle assembly of claim 1, further comprising a blister-type package comprised of a top web having a perimeter edge and a bottom web having a perimeter edge and comprising a pocket suitable for receiving the needle assembly therein, wherein the perimeter edge of the top web and the perimeter edge of the bottom web are sealed together along their respective lengths.

12. The needle assembly of claim 11, wherein the bottom web has an embossing area capable of receiving printed images.

13. The needle assembly of claim 11, wherein the top web comprises one or more of paper and a polymer film.

14. The needle assembly of claim 11, wherein the bottom web comprises one or more of paper and a polymer film.

15. A shielded, sterile, single-use bifurcated needle assembly for administering a vaccine comprising:
a hub including a tapered mating surface;
a bifurcated needle fixedly adhered to the hub and including two pointed prongs which are capable of penetrating or abrading the skin of a patient, and which are separated by a U-shaped channel which contains a unit dose of a vaccine; and
a packaging shield surrounding the bifurcated needle and removably attached to the tapered mating surface of the hub to form an air-tight seal between the hub and the packaging shield, thereby maintaining the bifurcated needle in a sterile environment within an interior of the packaging shield.

16. The bifurcated needle assembly of claim 15, wherein the hub includes means for attachment to a medical device.

17. The bifurcated needle assembly of claim 15, wherein the hub includes an internal luer taper.

18. The bifurcated needle assembly of claim 15, wherein the hub includes a profile for accommodating a user's fingers.

19. A method of packaging a single-use unit dose needle assembly comprising:
(a) providing a sheet of top web material, wherein the top web material comprises a plurality of rows, each row comprising a plurality of covers for a blister pack, each cover comprising a perimeter area;
(b) providing a sheet of bottom web material, wherein the bottom web material comprises a plurality of rows, each row comprising a plurality of bottoms for a blister pack, each bottom comprising a perimeter area and a pocket;
(c) providing a plurality of single-use unit dose needle assemblies comprising:
(i) a single-use unit dose needle including a prong end configured to hold a unit dose of a vaccine, and a handle end attached to a hub; and
(ii) a cylindrical needle shield having a closed end and an open end having a circumferential edge, the open end of the shield being capable of receiving the unit dose needle such that the circumferential edge securely engages a tapered surface of the hub;
(d) placing one of the single-use unit dose needle assemblies in (c) in each of the pockets in the bottom web material in (b); and
(e) sealing the perimeter areas in the top web material in (a) to the perimeter areas in the bottom web material in (b) to form a plurality of sealed single-use unit dose needle assembly packages.

20. The method of claim 19, wherein the bottom web (b) has an embossing area capable of receiving printed images.

21. The method of claim 19, wherein the top web material in (a) and the bottom web material in (b) comprises one or more of paper and a polymer film.

22. The method of claim 19, further comprising cutting each row of sealed single-use unit dose needle assembly packages from the plurality of sealed single-use unit dose needle assembly packages.
